# FASCICULE DE BREVET EUROPEEN

(11) **EP 0 629 242 B1**
(45) Date de publication et mention de la délivrance du brevet: **09.06.1999**
(21) Numéro de dépôt: 93905456.5
(22) Date de dépôt: 05.03.1993
(51) Int. Cl.: C12N 15/53, C12N 15/82, A01H 5/00

(54) **PROCEDE POUR ACCROITRE LA PRECOCITE D'UNE PLANTE ET/OU ABAISSER LA TENEUR EN NITRATES STOCKES DANS LA PLANTE**
METHODE ZUR ERHÖHUNG DER FRÜHREIFE BEI PFLANZEN UND/ODER REDUZIERUNG DES NITRATGEHALTES EINER PFLANZE
METHOD FOR ENHANCING PLANT PRECOCITY AND/OR REDUCING THE STORED NITRATE CONTENT OF A PLANT

(30) Priorité: 05.03.1992 FR 9202658
(43) Date de publication de la demande: 21.12.1994
(73) Titulaire: INSTITUT NATIONAL DE LA RECHERCHE AGRONOMIQUE, F-75007 Paris (FR)
(72) Inventeur: VINCENTZ, Michel, CEP 70875080s (BR); DORLHAC, François, F-92190 Meudon (FR); CHUPEAU, Yves, F-78550 Richebourg (FR); MOROT-GAUDRY, Jean-François, F-91190 Gif-sur-Yvette (FR); CABOCHE, Michel, F-78310 Maurepas (FR)
(74) Mandataire: Martin, Jean-Jacques
(86) Numéro de dépôt international: FR9300222
(87) Numéro de publication internationale: WO9318154

(56) Documents cités:
- EP-A- 0 227 909
- EP-A- 0 283 338
- EP-A- 0 303 780
- WO-A-91/04325
- PLANT MOLECULAR BIOLOGY. vol. 18, Janvier 1992, DORDRECHT, THE NETHERLANDS. pages 363 - 375 DORBE, M-F, ET AL. 'The tomato nia gene complements a Nicotina plumbaginifolia nitrate reductase deficient mutant and is properly regulated'
- THE PLANT JOURNAL vol. 1, no. 2, Septembre 1991, pages 267 - 274 NUSSAUME, L., ET AL. 'Constitutive nitrate reductase: a dominant conditional marker for plant genetics'
- EMBO JOURNAL vol. 10, no. 5, 1991, EYNSHAM, OXFORD GB pages 1027 - 1035 VINCENTZ, M., ET AL. 'Constitutive expression of nitrate reductase allows normal growth and development of Nicotiana plumbaginifolia plants' cité dans la demande
- DATABASE WPIL Section Ch, Week 8418, Derwent Publications Ltd., London, GB; Class C03, AN 84-107932 & DD,A,205 603 (GRAESER)

## Description

La présente invention concerne un procédé d'amélioration de la précocité de plantes, notamment des plantes supérieures. La présente invention concerne également un procédé d'abaissement de la teneur en nitrates stockés dans les plantes, notamment au niveau foliaire le cas échéant.

Les espèces cultivées ont un cycle de reproduction dont la durée limite souvent l'utilisation dans les régions septentrionales. En effet, la récolte de l'espèce doit pouvoir être effectuée avant le retour de mauvaises conditions météorologiques. Dans de nombreux exemples, la maturation des organes récoltés et des graines ne peut être obtenue en temps utile et rend nécessaire la récolte avant maturité, ou compromet la récolte. Ainsi de nombreuses espèces telles que le soja ne sont cultivées qu'au dessous de certaines latitudes pour cette raison. Par ailleurs des espèces déjà cultivées en zones septentrionales, ou en altitude, gagneraient à avoir un cycle plus court pour les mêmes raisons.

On remédie classiquement à ce type de problème, soit en utilisant des conditions de culture artificielles (culture en serre), méthode exploitable pour des cultures maraîchères, soit en sélectionnant pour une précocité accrue. Un gain de précocité peut être obtenu, soit en raccourcissant la durée de la phase de croissance végétative, soit en accélérant l'induction florale, soit enfin en facilitant la maturation des fruits ou graines à récolter. En général, la durée de la phase de croissance végétative apparaît contrôlée par un ensemble complexe de gènes, et se comporte comme un caractère quantitatif. Il n'y a pas d'indication d'un lien de causalité entre cette durée et un aspect particulier du métabolisme de la plante.

Le but de la présente est de fournir un procédé permettant de raccourcir la durée de la phase végétative, et donc d'obtenir un gain de précocité.

On entend donc, dans la présente demande comme dans le domaine technique de la présente invention, par "précoces" des variétés dont la durée entre la mise en terre de la graine et la récolte ultérieure est réduite. Une "précocité accrue" implique une durée de la phase de croissance de la plante plus brève qui conduit à une floraison et une maturation des fruits ou graines à récolter avancées dans le temps par rapport à la normale.

Un autre but de la présente invention était d'abaisser la teneur en nitrate de certaines plantes, notamment au niveau foliaire. Le taux élevé de nitrates peut en effet induire des risques pour la santé ainsi que des désagréments au plan des propriétés organoleptiques de certaines plantes, en particulier pour les épinards, la laitue, ou la carotte. C'est pourquoi les teneurs en nitrate, dans les plantes comestibles, sont maintenant réglementées dans de nombreux pays.

La Nitrate Réductase est une enzyme clé connue pour entrer en jeu dans la première étape de l'assimilation du nitrate dans les plantes.

Le nitrate est la plus importante source d'azote pour les plantes supérieures. Le nitrate est absorbé par les racines, transporté dans divers tissus de la plante, puis réduit en ammoniaque en deux étapes. La première étape exige l'enzyme Nitrate Réductase (NR) qui catalyse la réduction du nitrate en nitrite dans le cytoplasme. Dans une seconde étape, le nitrite est ensuite réduit dans le chloroplaste par la Nitrite Réductase. La réduction du nitrate est considérée comme une étape de contrôle majeure dans l'assimilation du nitrate et elle a été étudiée en détail chez les plantes supérieures (Wray, 1986). La NR est un homodimère portant trois cofacteurs, à savoir FAD, cytochrome b₅₅₇ et un cofacteur molybdoptérine (Campbell, 1988).

L'introduction dans une plante du gène de la NR a été proposée pour modifier les caractéristiques d'assimilation des nitrates par les plantes de manière prospective ou spéculative mais sans qu'aucune application concrète n'ait pu être effectivement trouvée jusqu'à ce jour.

Dans tous les cas, l'utilisation était toujours limitée à la modulation de l'assimilation des nitrates dans le temps, c'est-à-dire suivant le stade de développement de la plante, ou dans l'espace, c'est-à-dire pour favoriser l'assimilation au niveau des racines, des tubercules ou foliaire.

On a découvert de façon inattendue que la surexpression de la Nitrate Réductase dans les plantes transgéniques dans lesquelles un gène de NR a été introduit permettait d'une part d'abaisser de manière significative les teneurs en nitrate stockées sous forme de réserve dans la plante et, d'autre part se traduisait par une précocité plus grande, avec un gain de précocité de germination, une croissance accrue et une floraison plus précoce, c'est-à-dire un développement végétatif de la plante plus rapide qui la fait aboutir à floraison avec une avance d'environ deux semaines par rapport aux plantes témoins.

Une indicence de la surexpression de la Nitrate Réductase sur la précocité est inattendue. Il n'y a pas de travaux qui portent sur de telles études dans la littérature. Chez les céréales, une incidence éventuelle de la quantité de Nitrate Réductase sur les rendements a été étudiée par de nombreux auteurs (Clark, 1990). Toutefois, à l'occasion de ces études, il n'a pas été noté de relation évidente entre l'expression de l'enzyme et la précocité.

Les travaux récents de génétique n'ont pas non plus permis d'établir de relations directes entre la quantité de Nitrate Réductase exprimée au niveau foliaire et le transport ou le stockage du nitrate dans diverses espèces. L'étude de mutants déficients pour l'enzyme Nitrate Réductase de Nicotiana plumbaginifolia (Saux et coll. 1987) ou d'orge (Warner et Huffaker, 1989) a établi que ces mutants accumulent du nitrate au niveau foliaire à un niveau comparable aux plantes témoins capables d'assimiler le nitrate. La Nitrate Réductase n'est donc pas nécessaire au transport du nitrate. Par ailleurs, divers travaux d'étude du contrôle génétique de la teneur en nitrate, réalisés par Ostrem et Collins (1983) chez le tabac, et par Blim-Zandstra et Eenink (1986) chez la laitue ont abouti à la conclusion qu'il n'y avait pas non plus de corrélation nette entre la teneur en nitrate foliaire et la quantité de Nitrate Réductase foliaire. Cette absence de corrélation a été expliquée par le fait que la Nitrate Réductase étant elle-même inductible par le nitrate (Crawford. 1989), l'accumulation ou la réduction de la teneur en nitrate peuvent s'accompagner d'une augmentation ou d'une réduction corrélative de la quantité de cette enzyme dans les tissus, par effet rétroactif. Par contre, divers auteurs ont émis l'hypothèse que ce serait le besoin de la plante en osmoticum qui pourrait gouverner ses caractéristiques de stockage du nitrate, et non pas l'utilisation du nitrate par la Nitrate Réductase. La réduction de la teneur en nitrate et la précocité accrue des plantes qui surexpriment la Nitrate Réductase apparaissent donc inattendues.

Dans un cas comme dans l'autre, le mécanisme et les justifications théoriques manquent pour expliquer ces propriétés.

La présente invention a donc pour objet un procédé pour accroître la précocité d'une plante et/ou abaisser la teneur en nitrates stockés dans la plante, caractérisé en ce qu'on induit une surexpression de l'enzyme Nitrate Réductase dans la plante. En d'autres termes, on induit une surexpression de l'activité Nitrase Réductase dans la plante.

On entend ici par "Nitrate Réductase" (NR) une définition fonctionnelle qui inclut toute Nitrate Réductase capable de fonctionner comme un marqueur de sélection en conférant l'activité Nitrate Réductase à une cellule hôte déficiente en Nitrate Réductase. Cette définition inclut aussi toute Nitrate Réductase capable de fonctionner dans une plante donnée pour accroître l'activité Nitrate Réductase de ladite plante. Ce terme inclut donc non seulement l'enzyme spécifique de la plante spécifique à traiter mais toute autre enzyme Nitrate Réductase d'autres plantes, de microbes ou même d'autres espèces eucaryotes, si cette Nitrate Réductase est capable de fonctionner dans les plantes à traiter.

On entend par "surexpression" aussi bien une augmentation du taux d'activité de NR par rapport au taux exprimé dans une plante normale, qu'une dérégulation de l'expression conduisant à l'expression de l'activité NR dans un tissu ou à un stade de développement où celle-ci n'est normalement pas exprimée.

L'obtention de plantes exprimant de façon dérégulée la Nitrate Réductase peut être obtenue par différentes approches :
1) En sélectionnant des mutants de la plante à améliorer, mutants surexprimant la Nitrate Réductase ;
2) En introduisant par les méthodes du génie génétique un gène de Nitrate Réductase éventuellement modifié de façon appropriée à obtenir la modification de l'expression, et de préférence la surexpression de la Nitrate Réductase dans la plante à améliorer ;
3) En introduisant simultanément par les méthodes du génie génétique un gène de Nitrate Réductase et un gène de Nitrite Réductase éventuellement modifiés de façon appropriée à obtenir la modification de l'expression, et de préférence la surexpression dérégulée simultanée de ces deux enzymes dans la plante à améliorer.
4) En introduisant par les méthodes du génie génétique un gène de régulation de l'expression de la Nitrate Réductase et de la Nitrite Réductase éventuellement modifiés, de façon appropriée à obtenir la modification de l'expression, et de préférence la surexpression dérégulée conjointe de la Nitrate Réductase et de la Nitrite Réductase dans la plante à améliorer.

Ces plantes pourront être modifiées par les méthodes du génie génétique décrites dans la littérature, par transformation de cellules suivie de leur régénération, ou par transformation de tissus ou de gamêtes.

Dans un mode préféré de réalisation du procédé de l'invention, caracterisé en ce qu'on introduit dans le génome de la plante un gène étranger codant pour la Nitrate Réductase dans des conditions permettant son expression.

On entend par "gène fonctionnel codant pour la NR" une séquence d'ADN codant pour un polypeptide tel que défini ci-dessus comme "Nitrate Réductase", ladite séquence peut donc être plus courte ou plus longue que la séquence codante totale du gène complet de l'enzyme. En particulier, le "gène fonctionnel" pourra correspondre à une séquence codante partielle à savoir dépourvue des introns.

Le gène étranger est en général un gène hétérologue, c'est-à-dire qui provient d'un organisme d'une espèce différente que la cellule hôte, le gène codant pour un polypeptide ordinairement non produit par la plante dans le génome de laquelle il est introduit.

Le gène étranger introduit dans le génome de la plante peut aussi être un gène homologue au gène endogène c'est-à-dire dont l'expression produit la Nitrate Réductase ordinairement produite par la plante.

Par "conditions permettant son expression", on entend que le gène codant la Nitrate Réductase est placé sous le contrôle d'éléments assurant son expression.

En particulier, la séquence d'ADN codant pour la Nitrate Réductase est associée à une séquence régulatrice appropriée pour sa transcription et sa traduction (ci-après régulon) tels que des promoteurs, y compris des codons start et stop, des "enhancer", des opérateurs. Les moyens et méthodes pour identifier et sélectionner ces promoteurs sont bien connus de l'homme du métier.

Selon un autre mode de réalisation, on peut se contenter d'agir sur la régulation du gène endogène de la Nitrate Réductase en modifiant les gènes de régulation qui contribuent à son expression de manière à ce qu'ils induisent une surexpression de Nitrate Réductase endogène du fait de ces modifications, en particulier, on place le gène de la Nitrate Réductase sous le contrôle d'un promoteur hétérologue fort fonctionnel dans la plante transformée.

Par ailleurs, on a découvert selon la présente invention que le promoteur endogène des gènes de Nitrates Réductases de plante nécessite la présence de sucre pour être activé et induire l'expression de la Nitrate Réductase, la teneur en sucre dans la plante constitue dès lors un facteur limitant, en particulier aux faibles intensités lumineuses.

C'est pourquoi, avantageusement, lorsqu'on place le gène de la Nitrate Réductase endogène ou étranger sous le contrôle d'un promoteur hétérologue, de préférence, le promoteur hétérologue utilisé ne sera pas dépendant de la teneur en sucres.

Ainsi, le promoteur 35S du CaMV (Kay, Chan, Daly et McPherson, 1987), ou le promoteur du gène codant pour le facteur d'élongation de la traduction (Curie et coll. 1991), ou tout autre promoteur dont le fonctionnement ne dépend pas de la présence de sucre sont-ils utilisés avantageusement à cet effet. De même, de façon appropriée, des promoteurs inductibles par une carence en assimilats carbonés dérivés de la photosynthèse pourront être employés.

C'est pourquoi également, selon un autre mode de réalisation du procédé de l'invention, on se contente d'augmenter la teneur en sucre dans la plante pour favoriser l'expression de la Nitrate Réductase endogène sans introduire de gène étranger.

Comme gène hétérologue codant pour une Nitrate Réductase, on peut citer en particulier des gènes de plantes, notamment dicotylédones, comme le tabac (Vaucheret et coll. 1989), la tomate (Daniel-Vedele et coll. 1989), Arabidopsis (Crawford et coll. 1988), le haricot (Hoff, Stummann, et Henningssen, 1991) ou monocotylédones comme l'orge (Kleinhofs et coll. 1988) et le riz (Chol, Kleinhofs et An, 1989).

Il doit donc être bien entendu que l'invention implique non seulement l'utilisation des ADNc codant la Nitrate Réductase provenant notamment de plantes, mais aussi toute séquence d'ADN équivalente, c'est-à-dire qui diffère de la séquence d'ADNc seulement par une ou plusieurs mutations neutres, c'est-à-dire dont le changement ou la substitution de nucléotides en cause n'affecte pas la séquence primaire de la protéine résultante.

La présente invention implique aussi l'utilisation de séquences d'ADN complémentaires aux séquences mentionnées ci-dessus, en particulier qui présentent une homologie suffisante avec une séquence d'ADNc complémentaire de l'ARNm d'une Nitrate Réductase, de telle sorte qu'elles s'hybrident avec ladite séquence d'ADNc à 80% dans des conditions stringentes.

En fait, les Nitrates Réductases des différentes espèces dicotylédones présentent une grande homologie. Ainsi, la Nitrate Réductase de tomate présente environ 90% d'homologie avec la Nitrate Réductase de tabac et la séquence d'ADN codant la Nitrate Réductase de tomate présente une homologie de plus 80% (environ 81%) avec la séquence d'ADN codant la Nitrate Réductase de tabac.

Les Nitrates Réductases sont caractérisées en particulier par la présence des acides aminés invariants suivants (Daniel-Vedele, Dorbe, Caboche, et Rouzé, 1989) dans la séquence codante de l'enzyme (positions fournies par rapport à la séquence en acides aminés de la Nitrate Réductase de tabac) :
- Séquence CAGNRRKE des acides aminés 180 à 187 du domaine de fixation du cofacteur à molybdène de l'enzyme,
- Séquence HPGG des acides aminés 564 à 567 du domaine cytochrome b₅ de l'enzyme,
- Séquence GLP des acides aminés 677 à 679 du domaine cytochrome b₅ réductase de l'enzyme.

Les demandes de brevet EP 283 338 et EP 409730 décrivent des séquences d'ADN codant pour la Nitrate Réductase de tabac et de tomate respectivement.

Ledit gène fonctionnel codant pour la Nitrate Réductase peut être introduit dans des cellules de plantes selon des techniques connues. On pourra utiliser avantageusement dans ce cas, mais non obligatoirement le régulon constitutif du gène de la Nitrate Réductase.

On peut citer, tout d'abord, les méthodes de transfert direct de gènes telles que la micro-injection directe dans des cellules d'embryon de la plante (Neuhaus et Coll.. 1987) ou l'électroporation (Chupeau et Coll., 1988) ou encore la précipitation directe au moyen de PEG (Schocher et Coll., 1986) ou le bombardement par canon de particules (Mc Cabe et Coll., 1988)..

On peut aussi infecter la plante par une souche bactérienne notamment d'Agrobacterium tuméfaciens selon une méthode éprouvée (Schell et Van Montagu, 1983) ou d'Agrobacterium rhizogenes notamment pour les espèces récalcitrantes à la transformation (Chilton et Coll., 1982). La souche bactérienne comportera le gène codant pour la Nitrate Réductase sous le contrôle d'éléments assurant l'expression dudit gène. La souche pourra être transformée par un vecteur dans lequel est inséré le gène codant la Nitrate Réductase sous le contrôle d'éléments assurant l'expression dudit gène. Ce gène sera inséré par exemple dans un vecteur binaire tel que pBIN19 (Bevan, 1984) ou pMON 505 (Horsch et Klee, 1986) ou tout autre vecteur binaire dérivé des plasmides T1 et R1. Il pourra aussi être utilement introduit par recombination homologue dans un plasmide T1 ou R1 désarmé, tel que pGV 3850 (Zambryski et coll., 1983) avant la transformation de la plante.

A titre de vecteur d'expression comprenant le gène fonctionnel de la Nitrate Réductase selon l'invention, on peut citer les vecteurs comprenant une séquence d'ADN contenant au moins une origine de réplication telle que des plasmides, des cosmides, des bactériophages, des virus, etc. On utilisera de préférence toutefois des plasmides.

Lorsque l'on introduit un gène fonctionnel codant pour une Nitrate Réductase dans le génome de la plante, ce sera de préférence sous le contrôle de promoteur hétérologue.

A titre illustratif, on peut citer à cet effet des promoteurs constitutifs, comme celui du facteur d'élongation de la traduction (Curie et coll. 1991), des promoteurs tissus spécifiques comme celui de la patatine exprimé dans les tubercules (Rocha-Sosa et coll., 1989), ou comme celui de la petite sous-unité de la ribulose-bis-phosphate décarbosylase exprimés dans les feuilles (Thomson et White 1991), ou dérivés de virus végétaux comme le promoteur 35S du virus de la mosaïque du chou-fleur ou CaMV (Kay, Chan, Daly et McPherson, 1987) ou dérivés de l'ADN-T des Agrobactéries ou toute source de promoteur fonctionnel dans la plante transformée.

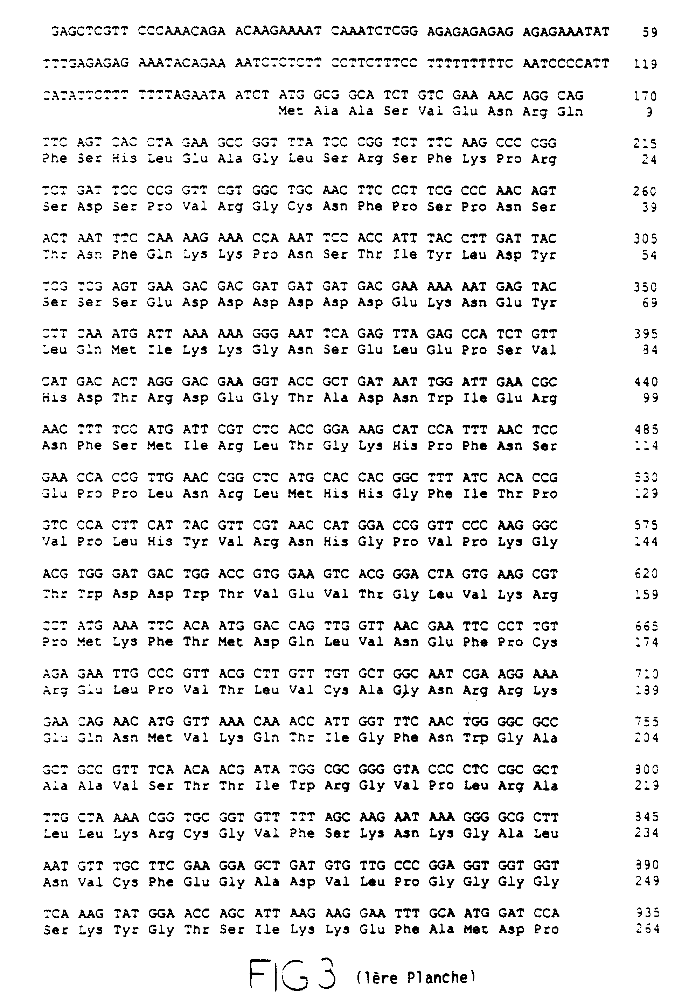

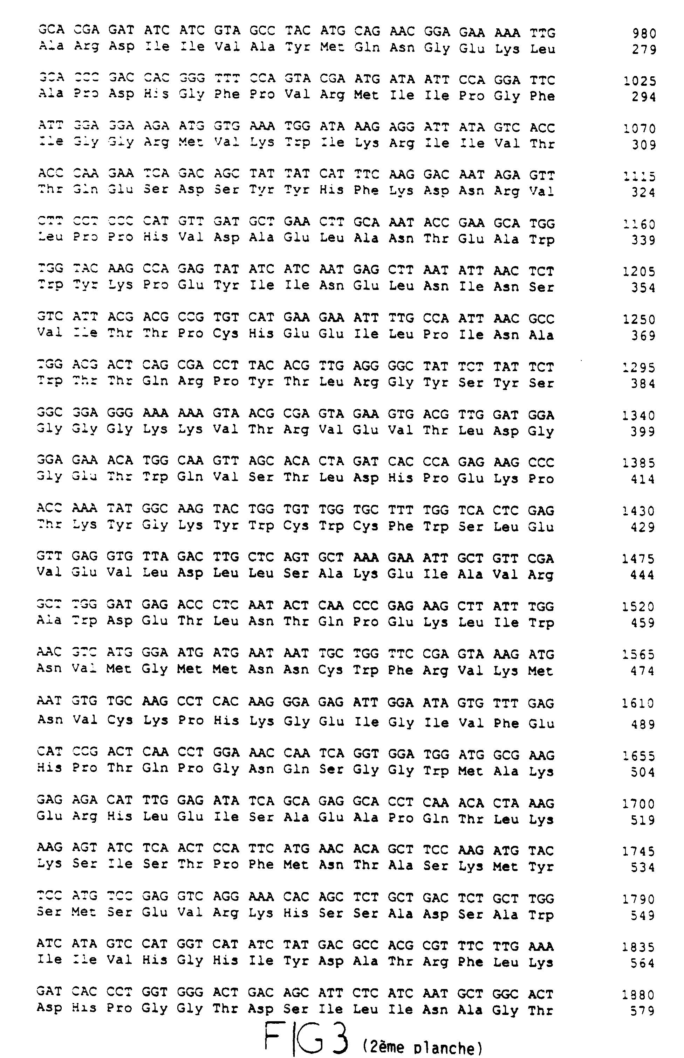

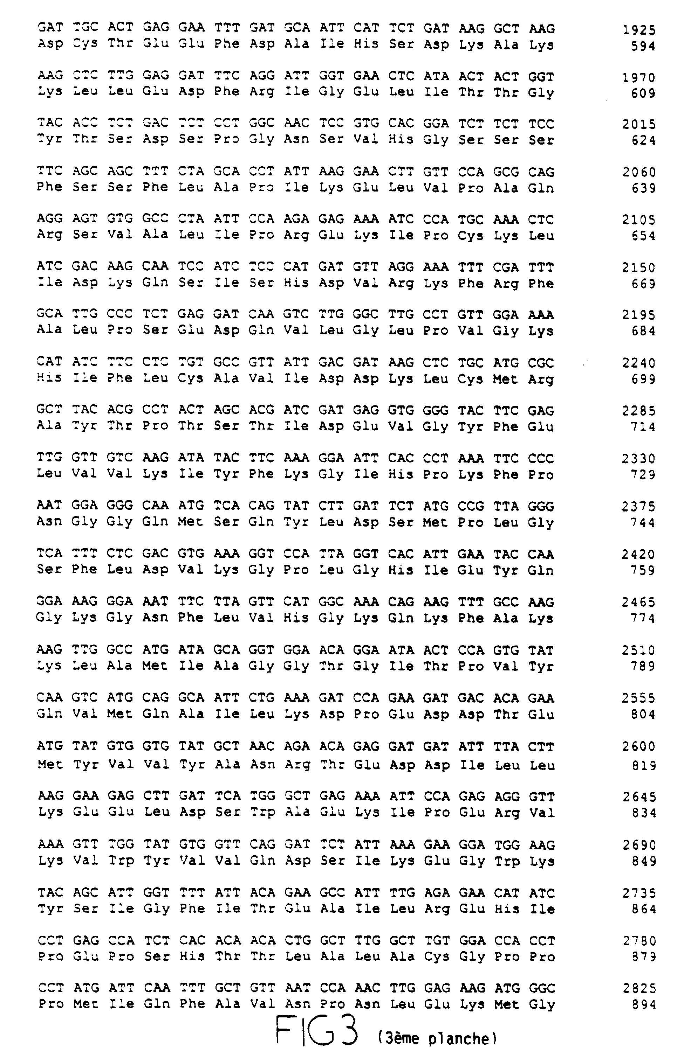

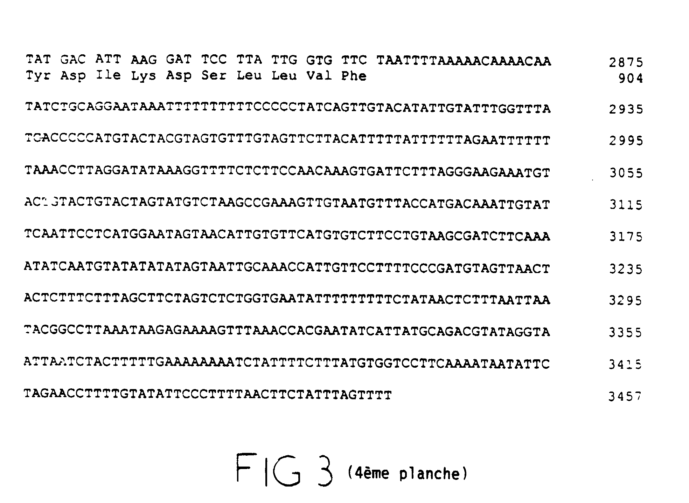

Dans les exemples ci-après, donnés à titre purement illustratif, le gène étranger codant pour la Nitrate Réductase est dérivé du gène Nia 2 de la Nitrate Réductase du tabac décrit dans la Figure 3 ( Vaucheret et coll., 1989) et le gène étranger codant pour la Nitrate Réductase est inséré dans le plasmide pBin 19.

Les caractéristiques des plantes exprimant de manière dérégulée la Nitrate Réductase conjointement ou non avec l'expression dérégulée de la Nitrite Réductase obtenues par le procédé selon l'invention sont un gain de précocité de germination, une croissance accrue et une floraison plus précoce.

En outre, cette expression dérégulée se traduit par des caractéristiques supplémentaires telles que :
- une baisse de la teneur en nitrate dans les tissus de la plante, et
- de conférer aux plantes obtenues une caractéristique qui permet de les distinguer aisément des plantes non modifiées du fait de la sensibilité accrue au chlorate des plantes modifiées.

Le chlorate est utilisé comme défoliant. Un traitement au chlorate s'avère utile en préalable à la récolte de certaines plantes à maturité comme le coton.

Ainsi, on peut, grâce au procédé de l'invention, utiliser le chlorate à des doses moins importantes pour induire la défoliation des plantes modifiées selon l'invention si ce traitement s'avère utile.

D'autres caractéristiques et avantages de la présente invention apparaîtront à la lumière de la description détaillée du mode de réalisation suivant.

La Figure 1 représente l'étude comparée de la croissance de la descendance du tabac transformé 3051 PBD6, cultivée in vitro. Le nombre moyen de feuilles par plantule (Ordonnée) a été mesuré et représenté en fonction du nombre de jours écoulés après le semis (Abscisse).

La Figure 2 représente la croissance en serre et floraison de plantes surexprimant ou non la Nitrate Réductase. Ces plantes ont été repiquées en serre au même stade de développement et cultivées dans les mêmes conditions expérimentales.

La Figure 3 représente la séquence du gène dérivé de Nia2 de la Nitrate Réductase de tabac privé de ses introns.

La Figure 4 représente la teneur en ions nitrate des différents étages foliaires de la lignée 30.51.2 PB D6. Les plantes ont été cultivées en champ en présence de 400 kg d'azote à l'ha.

La Figure 5 représente la teneur en ions nitrate des différents étages foliaires de la lignée 34.2.5 BB 16. Les plantes ont été cultivées en champ en présence de 400 kg d'azote à l'ha.

### EXEMPLE 1

### Effet de la surexpression de la Nitrate Réductase sur la précocité du tabac

Des gènes recombinants dérivés du gène de la Nitrate Réductase de tabac (Brevet Européen EP 283 338) ont été initialement réalisés selon une procédure classique décrite par Vincentz et Caboche (1991) afin de complémenter des mutants de N. plumbaginifolia déficients pour la Nitrate Réductase. Ces gènes sont constitués de la manière suivante. La séquence codante de la Nitrate Réductase (ADNc dérivé du messager d'origine Nia2) précédée ou non de la séquence 5' non traduite de ce transcrit, et suivie de séquences d'arrêt de la transcription dérivée d'un des gènes de la Nitrate Réductase du tabac, ou du CaMV a été placée sous le contrôle du promoteur fort de l'ARN 35S du CaMV.

Dans le présent exemple, ce gène a été inséré dans un plasmide vecteur binaire pBin19 (BEVAN, 1984) et introduit selon une procédure classique dans le génome de tabacs industriels, variétés PBD6 et BB16, par l'intermédiaire d'Agrobacterium tumefaciens (souche LBA 4404) (Hoekema et al., 1983). La transformation a été réalisée par inoculation de disques foliaires d'une surface moyenne de 5 cm². Le plasmide pBin19 portant le gène NPTII (néomycine phosphotransférase) conférant la résistance à la kanamycine après transformation, les transformants ont été sélectionnés pour leur aptitude à se développer sur une dose de 100 mg/l de cet antibiotique. Sur un total de 125 explants inoculés pour la variété BB16, et 190 explants pour la variété PB D6, le nombre de plantes transformées obtenues s'élève respectivement à 10 et 281. Parmi ces plantes, certaines peuvent atteindre un niveau d'activité Nitrate Réductase six fois supérieur à celui observé pour le type sauvage. Les caractéristiques de germination et de croissance de deux transformants qui surexpriment la Nitrate Réductase ont été présentées ici et sont représentatives.

### Etude de la croissance in vitro de la descendance du transformant 30.51

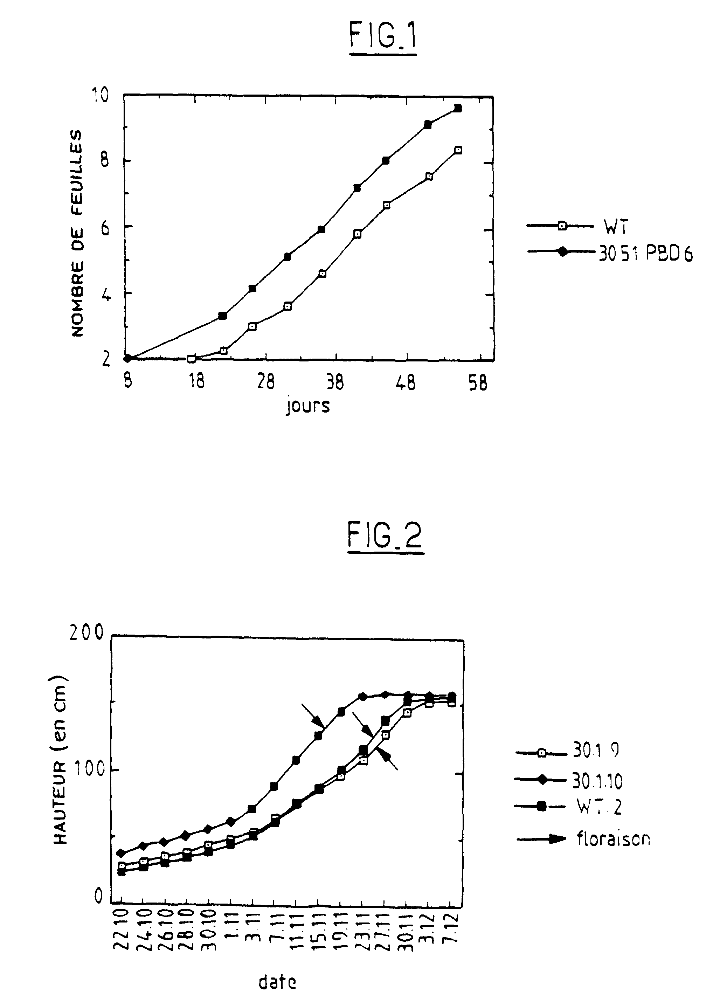

Ce transformant obtenu à partir du génotype PBD6, surexprime approximativement 600% du niveau de la Nitrate Réductase du témoin non transformé. Sa descendance a été récoltée et étudiée. Après stérilisation en surface pendant 40 minutes dans une solution de 800 ml d'eau contenant un comprimé de Bayrochlore de 1 ml de Teepol, puis un rinçage par trois fois dans 800 ml d'eau stérile, les graines ont été semées sur du milieu de bouturage contenant 20 mM de nitrate. 12 graines de cette plante, ainsi qu'un nombre identique du type sauvage ont ainsi été cultivés in vitro dans des tubes, puis placés dans des chambres de culture dont la température est maintenue à 25°C et le degré hygrométrique à 65% ; l'éclairage de ces chambres est assuré par des tubes fluorescents type "blanc industrie" Philips 40W, assurant une intensité lumineuse de 60µE m⁻²s⁻¹, 16 heures par jour. La Figure 1 montre que la descendance du transformant germe significativement plus précocement que celle du témoin non transformé. Une moyenne effectuée sur chaque lot révèle que la descendance de la plante transformée germe 9 jours avant celle du type sauvage.

### Etude de la croissance de la descendance du transformant primaire 30.1 BB16

Les graines issues de la plante 30.1 BB16 (transformant primaire surexprimant le gène de la Nitrate Réductase à un niveau trois fois supérieur au témoin non transformé) ont été semées sur de la tourbe et alimentées par la solution nutritive de Coïc et Lesaint, contenant 20 mM de nitrate comme source azotée. 10 plantes ont ainsi été disposées en serre sous un éclairage de 16 heures par jour. Il ressort de leur étude (Figure 2) que les plantes surexprimant constitutivement le gène de la Nitrate Réductase (telles que la plante 30.1.10 prise comme exemple) voient leur floraison accélérée de 10 jours par rapport aux plantes du type sauvage (WT.2 et 30.1.9, cette dernière plante étant une plante du type sauvage ne surexprimant pas la Nitrate Réductase et ne résistant pas à la kanamycine, ayant ségrégé dans la descendance du transformant primaire).

### EXEMPLE 2

### Etude de la sensibilité au chlorate des plantes transgéniques exprimant constitutivement le gène de la nitrate réductase

Des semis en terrine de descendants homozygotes du transformant 30.51PBD6 ont été effectués parallèlement à des semis témoins. Ces terrines ont été arrosées avec une solution contenant 0.5 mM, 1,5 mM, 5 mM ou 10 mM de chlorate de potassium dix jours après le semis, au stade deux feuilles. Alors que les plantes témoins manifestent les symptomes de chlorose puis de brûlure foliaire caractéristiques de l'effet du chlorate uniquement aux doses de 5 et 10 mM, les plantes transgéniques sont tuées dès la dose la plus faible de chlorate employée (0.5 mM).

### EXEMPLE 3

### Effet de la surexpression de la Nitrate Réductase sur la teneur foliaire en nitrate chez Nicotiana plumbaginifolia

La séquence codante de la Nitrate Réductase (ADNc dérivé du messager d'origine Nia2) précédée de la séquence 5' non traduite de ce transcrit, et suivie de séquences d'arrêt de la transcription dérivées du gène Nia2 de la Nitrate Réductase du tabac a été placée sous le contrôle du promoteur fort de l'ARN 35S du CaMV. Dans le présent exemple, ce gène a été inséré dans un plasmide vecteur binaire pBin19, et introduit dans le génome du mutant E23 de Nicotiana plumbaginifolia déficient pour le gène de structure de la Nitrate Réductase selon une procédure classique décrite dans l'exemple 1. Un transformant, C1, exprimant une activité Nitrate Réductase de 29 nM de nitrite par minute et par mg de protéines totales, soit 178% du témoin sauvage a été étudié. Des plants de Nicotiana plumbaginifolia sauvages ou du transformant C1 ont été cultivés en serre. à l'I.N.R.A. de Versailles au cours de l'automne 1990.
a) Au cours du premier essai (7/09 au 7/11/90), les plantes ont été placées sur un mélange tourbe/argile et arrosées 2 fois par 24 h par une solution nutritive complète nitroco-ammoniacale (430 ml par plante et par jour), contenant soit 10,2 mM de nitrate et 1,8 mM d'ammonium soit 15,3 mM de nitrate et 2,7 mM d'ammonium. L'éclairement naturel a été complémenté par un éclairage d'appoint assurant de l'ordre de 100 mmol m⁻² s⁻¹ PAR pendant 16 h (lampes phytoclaude). La récolte a eu lieu au stade début floraison. Des analyses ont été pratiquées sur 4 plantes prises au hasard parmi les 28 plantes cultivées, par génotype et par type de condition de culture.
b) Au cours du second essai (25/10/90 au 30/01/91), les plantes ont été placées sur sable inerte et arrosées toutes les 15 minutes pendant 24 h, par une solution nutritive complète (9,6 l par plante et par jour) contenant soit I mM de nitrate seul, soit 12 mM de nitrate seul. L'éclairement naturel a été complémenté par un éclairage d'appoint identique à celui de l'essai précédent mais pendant 12 h. Les plantes ont été récoltées au stade rosette, les analyses ont été pratiquées sur un échantillon moyen regroupant 4 plantes prises au hasard parmi les 14 plantes cultivées, par génotype et par type de condition de culture.

Les analyses pratiques après récolte montrent les tendances suivantes (voir tableau ci-joint) :
1) La teneur en nitrate est beaucoup plus faible chez les plantes transformées que chez les plantes témoins (de -30% à -70%) ;
2) La teneur en azote réduit est plus élevée chez les plantes transformées. Il est à noter qu'il existe un seuil maximal correspondant à 4,5% d'azote réduit chez tous les types de plantes quel que soit le type de nutrition azotée ;
3) La teneur en azote protéique est la même chez les plantes transformées et témoins ;
4) La teneur en azote total chez les plantes transformées est légèrement plus faible que chez les plantes témoins ;
5) Le type de nutrition azotée (en quantité et en qualité) modifie la teneur en composés azotés par plante mais ne semble pas avoir d'effet sur le comportement général des plantes.

En conclusion, les plantes tranformées accumulent beaucoup moins d'azote sous forme nitrique que les plantes témoins. Les plantes transformées accumulent l'azote sous forme réduite. Cet excès d'azote réduit interne pourrait être l'une des causes de la plus forte teneur en matière sèche (M.S.) ainsi que de la moindre production de biomasse fraîche et sèche (de -15% à -30%) observées chez les plantes transformées.

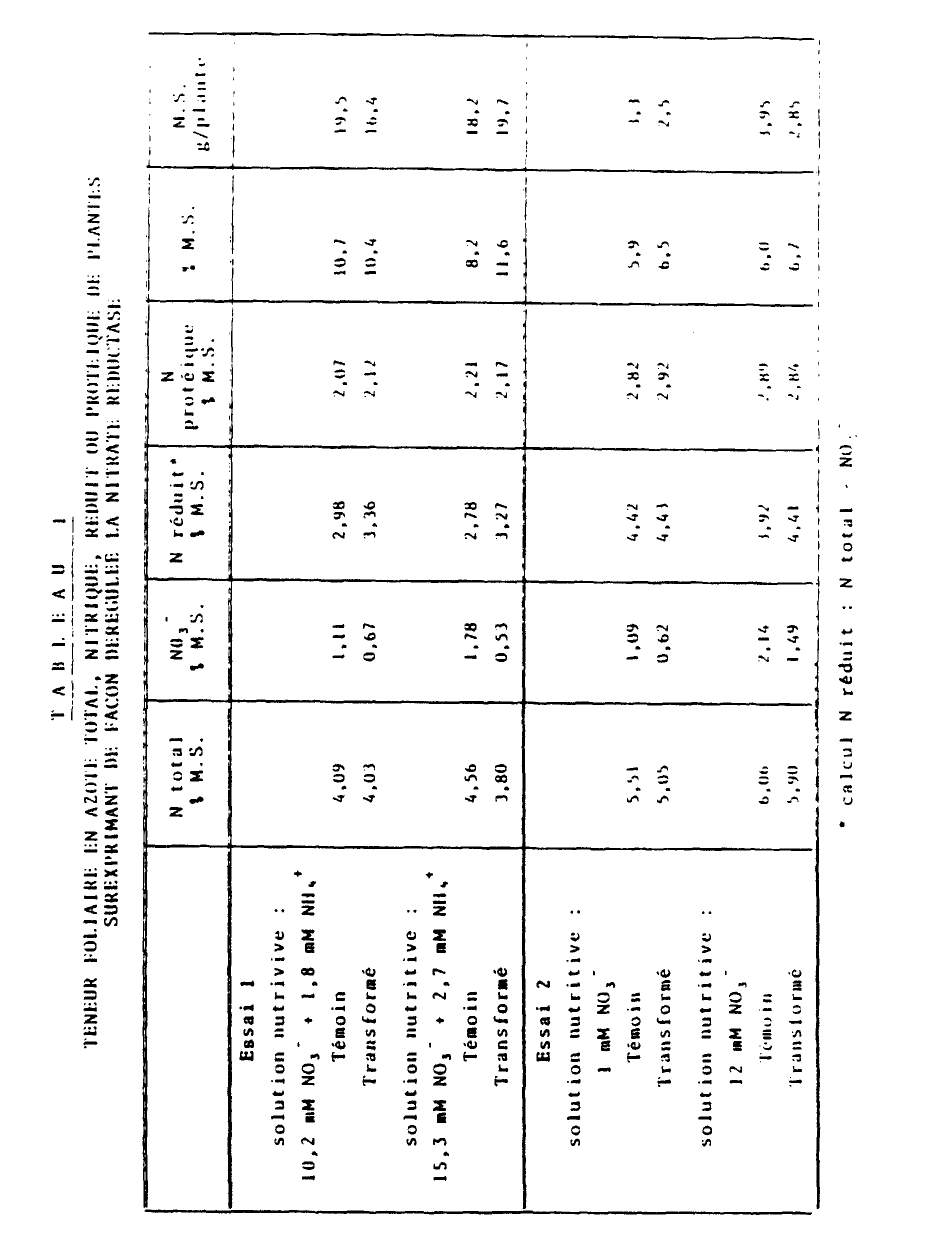

### EXEMPLE 4

### Rôle du sucre dans l'expression de la Nitrate Réductase

Des plantes de Nicotiana plumbaginifolia sauvages et CI identiques à celles décrites dans l'exemple 3 ont été repiquées au stade 4 feuilles, cultivées trois semaines en serre, à l'I.N.R.A. de Versailles au cours de l'automne 1990 dans un terreau arrosé par une solution nutritive complète nitroco-ammoniacale, contenant 12 mM de nitrate et 2 mM d'ammonium. Les plantes ont été transférées dans une chambre de culture pendant 6 jours et soumises à une photopériode de 16 heures à 25°C, avec un éclairement de 130 mE m⁻² s⁻¹ PAR pendant 16 h (lampes phytoclaude) suivi de 8 heures d'obscurité à 16°C. Les plantes ont ensuite été placées à l'obscurité durant 72 heures, tout en restant alimentées par la solution nutritive. A ce stade, des analyses ont été pratiquées sur les feuilles de 4 plantes prises au hasard par génotype témoin ou C1. Le niveau de transcrit Nitrate Réductase a été mesuré par la méthode de northern (Thomas. 1980) dans ces feuilles. Cette quantité de transcrit décroît d'un facteur 20 à 50 dans les plantes témoins placées à l'obscurité, alors qu'elle décroît seulement de 50% dans les plantes C1, placées dans les mêmes conditions. Les feuilles prélevées sur ces plantes témoin, maintenues à l'obscurité et dont le pétiole est plongé durant quatre heures dans une solution de conservation (Chlorure de potassium 40 mM, et chlorure de calcium 10 mM) contenant 0,2% de glucose accumulent à nouveau du transcrit Nitrate Réductase à un niveau approximatif de 25% par rapport aux conditions initiales précédant le transfert à l'obscurité. Par contre, cette accumulation n'est pas observée pour des feuilles témoins maintenues à l'obscurité dont le pétiole est plongé dans la solution de conservation sans glucuse. Dans les plantes CI, le niveau de transcrit Nitrate Réductase reste élevé aux diverses étapes de l'expérience, montrant que la réduction du niveau de transcrit Nitrate Réductase dans les plantes témoins ne résulte pas d'un ralentissement général du métabolisme dû à la carence en sucre. La teneur en sucre apparaît donc comme un signal important pour l'expression du gène non modifié de la Nitrate Réductase, et peut donc constituer un facteur limitant de cette expression aux faibles intensités lumineuses.

### EXEMPLE 5

### Effet de la surexpression de la Nitrate Réductase sur la teneur foliaire en nitrate chez Nicotiana tabacum

Au cours d'un essai, les plantes ont été disposées en trois blocs, comprenant chacun 8 parcelles de 48 plantes ayant reçu pour apport en azote des doses de 200 ou 400 kg par ha. Les plantes ont été écimées après la floraison. Le plan d'expérimentation par bloc a été déterminé aléatoirement, et 10 pieds par parcelle, pris au hasard, ont fait l'objet de caractérisations chimiques.

### Méthodes de dosage

### Ions nitrate

Le dosage est réalisé sur un appareil à flux continu de type Technicon autoanalyser AA Il C. 500 mg de tabac lyophilisé sont disposés dans 120 ml d'eau et agités pendant 30 min. La suspension est filtrée après que le volume ait été ajusté à 200 ml. L'échantillon prélevé est réduit en ions nitrite sur une colonne de cadmium puis est mélangé avec du réactif de Gness (sulfanilamide 10 g/l : acide phosphonque concentré 10% : N-naphtyl-éthylène-diamine 0,5 g/l). Après coloration, la densité optique est lue à 560 nm et la teneur en ions nitrate est déterminée par référence à une gamme étalon réalisée à l'aide de nitrate de potassium.

### Ions nitrite

Les teneurs en ions nitrite sont évaluées de manière analogue à celle utilisée pour le dosage des ions nitrate sur un appareil de type technicon autoanalyseur AA II. 500 mg de poudre de tabac sont disposés dans 50 ml de solution d'extraction (KCI 1% ; sulfanilamide 0,5% ; triton X100 0,2%) et agités 30 min. La solution est filtrée sur papier Whatman, 10 ml du filtrat sont alors mis en présence de 500 mg de charbon actif afin de décolorer les extraits de tabac. L'échantillon est alors coloré par le réactif de Gness décrit précédemment. L'absorbance est lue à 560 nm et la teneur en ions nitrite est déterminée par référence à une gamme étalon.

### Azote total

La minéralisation de la matière organique est réalisée dans un four à la température de 420°C. 500 mg de poudre de tabac sont digérés par 15 ml d'acide sulfunque concentre en présence de 1 g de catalyseur comprenant du sélénium (0.2 partie), du sulfate de cuivre (1 partie) et du sulfate de potasse (1 partie). Après 1 h dans le four, l'azote passe sous forme de sulfate d'ammonium. 50 ml d'eau sont alors ajoutés et l'ammoniaque fonné est libéré par un excès de lessive de soude à 30%, puis distillé par entraînement à la vapeur dans un appareil semi-automatique Técator. L'ammoniaque est recueilli dans 20 ml d'acide borique à 2% et titré avec de l'acide sulfurique 0.05 N.

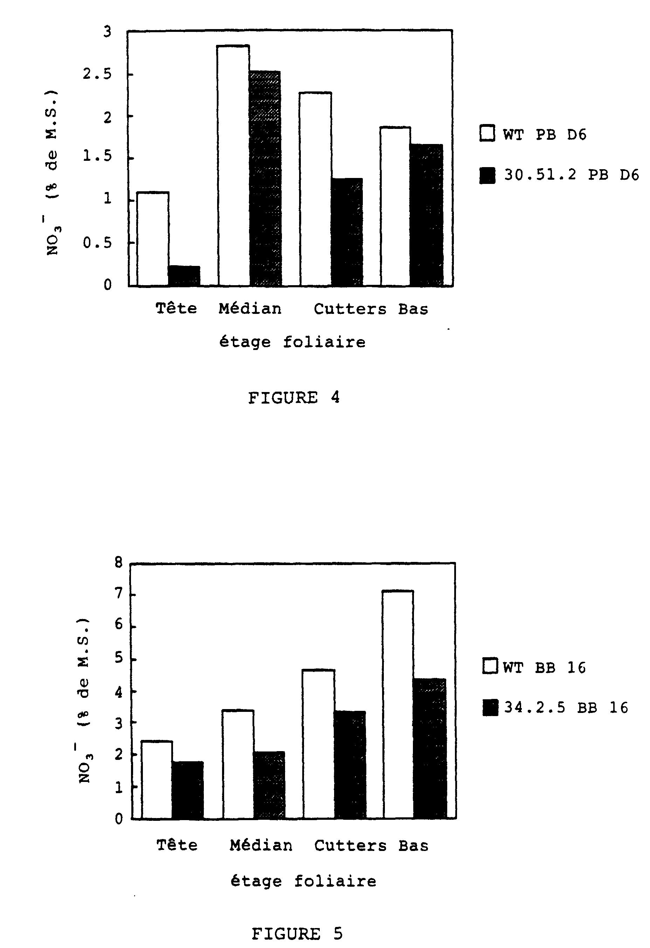

Les analyses après récolte montrent les tendances suivantes (voir figures 4 et 5, et tableau 2) :
1) Chez le type sauvage, l'effet d'une forte dose d'azote (400 kg/ha) est très net sur la croissance, mais les ions nitrate, malgré l'écimage, restent stockés dans les feuilles (*tab.*2). L'accumulation d'ions nitrate est d'autant plus importante chez BB 16, que cette variété est un mutant déficient chlorophyllien.
2) La teneur en ions nitrate des cutters, représentative de l'accumulation chez le tabac, baissent significativement de 38% pour un tel apport en azote, alors que la baisse pour la lignée 34.2.5 BB 16 atteint 28%. Chez la lignée 30.51.2 PB D6 (*fig*.4). ces diminutions s'étalent selon les étages foliaires de 11% à 75% pour une baisse globale de 36%, alors que pour la lignée 34.2.5 BB 16 (*fig.*5), ces baisses varient de 26% à 39% pour une valeur globale de 33%. La diminution de la quantité d'ions nitrate accumulée dans la feuille est toutefois plus importante chez la lignée 34.2.5 BB 16 que chez la lignée 30.51.2 PB D6, cette variété accumulant relativement peu les ions nitrate.
3) Les ions nitrite s'ils sont nombreux dans les feuilles hautes chez la lignée 34.2.5 PB D6, diminuent dans les feuilles de la base (*tab.* 2). D'autre part, chez la lignée 30.51.2 PB D6, l'épuisement des ions nitrate semble être à l'origine de la baisse des ions nitrite. L'accumulation prévisible d'ions nitrite consécutive à la dérégulation du gène de la NR est donc limitée, et a tendance à s'estomper à maturité.
4) La richesse en azote des feuilles dépend directement de l'apport en azote initial, mais on peut remarquer que chez les individus trangéniques, l'application de la dose la plus forte (400 kg d'azote/ha) est sans effet significatif sur la teneur en azote total (*tab*. 2) .Ce n'est seulement que sur la dose la plus faible (200 kg d'azote/ha) que l'on observe une différence (*tab.* 2). La hausse de la teneur en azote total, qui est plus forte dans les feuilles de tête, se limite à 19% pour la lignée 30.51.2 PB D6 et 31% pour la lignée 34,2.5 BB 16. Bien que ce bilan soit limité aux feuilles, il semble tout de même que l'absorption d'azote soit plus importante.

En conclusion. les plantes transformées accumulent moins d'azote sous forme nitrique que les plantes témoins. L'assimilation de l'azote chez les plantes transformées est plus efficace, et elles accumulent l'azote sous forme réduite.

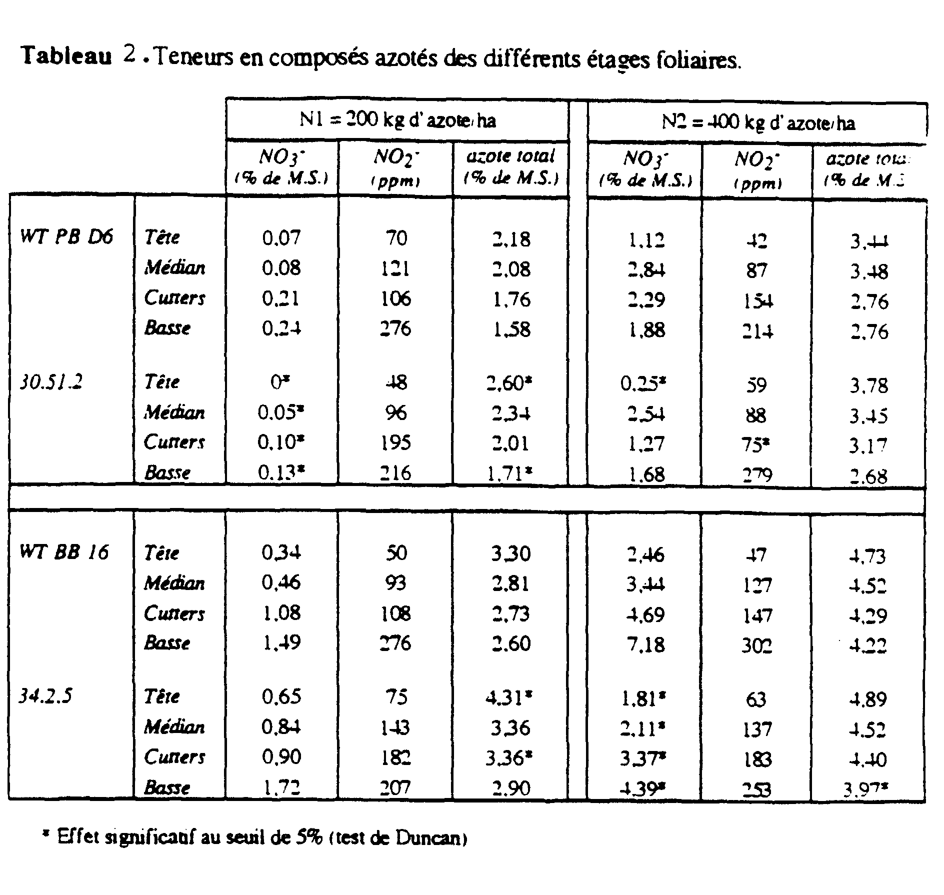

### EXEMPLE 6

### Production de laitues (Lactuca sativum) transformées

La teneur en Nitrate dans la laitue est très élevée et dépasse les niveaux acceptables pour la consommation si l'éclairement naturel de la culture est limité, comme c'est le cas dans les serres pendant l'automne, l'hiver et le printemps.

Une souche d'Agrobactérium tumefaciens (LBA-4404) contenant un plasmide vecteur binaire dérivé de pBinl9 (pBCSL16), contenant le gène de la Nitrate Réductase de tabac et le gène nptII (néomycine phosphotransférase) a été utilisée pour la production de l'étude transformée. Le gène de tabac était placé sous le contrôle du même promoteur 35S. Les constructions utilisées sont identiques à celles de l'Exemple 1. Quatre variétés de laitues de serre (Flora, Cortina, Luxor et Evola) ont été utilisées pour les expériences de transformation. Après une culture des explants blessés et des Agrobactéries (cf. Michelmore et al. PI. Cell Rep. 6:439 (1987)), plusieurs bourgeons ont été régénérés par calogenèse sur une culture sélective (100 mg/l de kanamycine). On a vérifié l'état transformé des bourgeons indépendants par un dosage de l'activité npt Il. Plusieurs des plantes transformées ont été ensuite transférées dans une serre.

### Etude de l'activité Nitrate Réductase et de la teneur de nitrate foliaire chez la laitue transformée (génération Ro)

Les plantes transformées ont été élevées dans une serre dans des conditions favorables. Parmi les plantes, on observe des phénotypes variables.

Trois semaines après le transfert à la serre, les plantes ont été ombragées (approximativement 1.000 lux) pendant deux jours. Ensuite, des échantillons de feuilles (disques de 2 cm de diamètre) ont été prélevés pour une déterminationde l'activité Nitrate Réductase selon une méthode connue (cf. Blom. Zandstra, Lamp Plant. Nutr., n° 6-611 (1983)). Le tableau 3 montre que, parmi les 104 plantes transformées, environ 13% montrent une activité très élevée en Nitrate Réductase (plus de 400%) en comparaison avec des plantes témoins régénérées in vitro. Pour une vingtaine de transformants primaires. la teneur en nitrate a été mesurée selon une procédure classique décrite par Sen Donaldson (J. Assoc. Off. Anal. Chem. 61 : 1389 (1978)).

En général, la teneur en nitrate était réduite de près de 50% chez des plantes avec une activité Nitrate Réductase très élevée. Pour la plus grande part, l'activité npt II était très élevée dans ces plantes.

**TABLEAU 3 :**

| Nombres de plantes de laitue transgénique primaire (Ro) et son activité Nitrate Réductase (NR) mesuré in vivo | | | | |
|---|---|---|---|---|
| | Nombre de plantes avec une activité NR⁽¹⁾ | | | |
| Génotype | 200% | 200-400% | 400% | Total |
| Flora | 11 | 5 | 1 | 17 |
| Luxor | 24 | 9 | 7 | 40 |
| Cortina | 8 | 3 | 2 | 13 |
| Evola | 24 | 7 | 3 | 34 |
| Total | 67 | 24 | 13 | 104 |

| | | | | |
|---|---|---|---|---|
| (1) Activité relative en comparaison avec le niveau des témoins non transformés. | | | | |

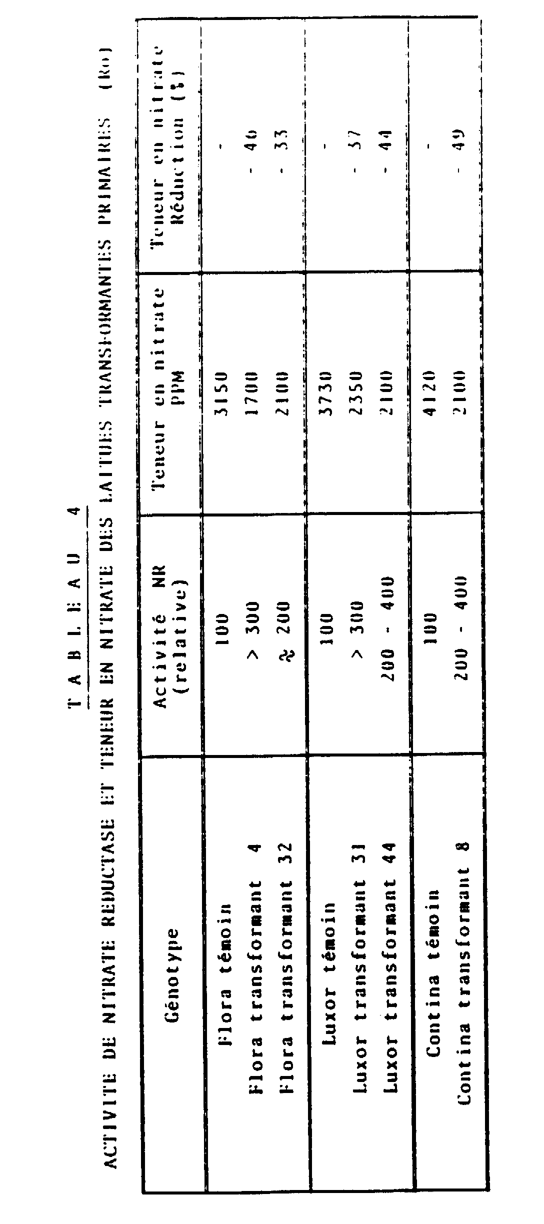

La Figure 3 représente une séquence identifiée comme ci-dessous :
Type de séquence : Nucléotide et sa protéine correspondante
Longueur de la séquence : 3457 paires de bases
Nombre de brins : simple
Configuration : linéaire
Type de molécule : ADN complémentaire (ADNc)

### ORIGINE

Organisme : Plante, Nicotiana tabacum
Source expérimentale : feuilles
Nom de la lignée : N. tabacum cv. Xanthi XHFD8

### CARACTERISTIQUES

de 1 à 143 paires de bases : séquence 5' non traduite (leader)
de 144 à 2855 paires de bases : séquene codante pour l'apoenzyme nitrate réductase
de 2856 à 3457 paires de bases : séquence 3' non traduite

### PROPRIETES

ADNc du messager codant pour l'apoenzyme de la nitrate réductase

### Bibliographie

BEVAN M. - 1984 - Binary Agrobacterium vectors for plant transformation Nucleic Acid Res. 12 : 8711-8712.

BLOM-ZANDSTRA, M. AND EENINK, A.H. - 1986 - Nitrate concentration and reduction in different genotypes of lettuce J. Amer. Soc. Hort. Sci 111 : 908-911.

CAMPBELL 1988 - Higher plant Nitrate Reductase Curr. Top. Plant Biochem. Physiol. 7,1-15.

CHOI, H., KLEINHOFS A., AND AN, G. (1989) Nucleotide sequence of rice nitrate reductase genes. Plant Molec. Biol. 13 : 731-733.

CHUPEAU M.C., BELLINI C., GUERCHE P., MAISONNEUVE B., VASTRA G.. CHUPEAU Y. (1989) Transgenic plants of lettuce (Lacuca sativa) obtained through electroporation of protoplasts. Bio/technology 7 : 503-508.

CLARK R.B. Physiology of cereals for mineral nutrient uptake, use, and efficiency, in "Crops as enhancer of nutrient use" Baligar V.C. and Duncan R.R. Eds Academic press, San Diego, London (1990) pp 131-210.

CRAWFORD N. AND DAVIS R.W. Molecular analysis of nitrate regulation of nitrate reductase in squash and Arabidopsis, in "Molecular and genetic aspects of nitrate assimilation" Wray J. and Kinghorn J.R. Eds Oxford Science Publications, Oxford, New York, Tokyo (1989) pp 328-337.

CRAWFORD, N., SMITH, M., BELLISSIMO, AND DAVIS, R.W. (1988) Sequence and nitrate regulation of the Arabidopsis thaliana mRNA encoding nitrate reductase, a metalloflavoprotein with three functional domains. Proc. Natl. Acad. Sci. USA 85 : 5006-5010.

CURIE C., LIBOZ T., BARDET C., GANDER E., MEDALE C., AXELOS M., LESCURE B. (1991) Cis and trans-acting elements involved in the activation of Arabidopsis thaliana Al gene encoding the translation elongation factor EF-la Nucleic Acid Res. 19 : 1305-1310.

DANIEL-VEDELE, F. DORBE, M.F., CABOCHE, M., AND ROUZE, P. (1989) Cloning and analysis of the nitrate reductase gene from tomato : a comparison of nitrate reductase protein sequences in higher plants. Gene 85 : 371-380.

HOEKEMA A., HIRSCH P.R., HOOYKAAS P.J.J. et SCHILPEROORT R.A.-1983 - A binary plant vector strategy based on separation of vir- and T-region of the Agrobacterium tumefaciens Ti-plasmid. Nature, 303, 179-180.

HOFF, T., STUMMANN, B.M., AND HENNINGSSEN, K.W. (1991) Cloning and expression of a gene encoding a root specific nitrate reductase in bean (Phaseolus vulgaris). Physiol. Plant; 82 : 197-204.

HORSH R.B. AND KLEE H.J. (1986) Rapid assay of foreign gene expression in leaf discs transformed by Agrobacterium tumefaciens Proc. Natl. Acad. Sci. USA 83 : 4428-4432.

KAY R., CHAN A., DALY M., McPHERSON J.(1987) Duplication of CaMV 35S promoter sequences creates a strong enhancer for plant genes Science 236 : 1299-1302.

KLEINHOFS, A., WARNER R. L., HAMAT, H.B., JURIDEK, M., HUANG. C., AND SCHNORR, K.(1988) Molecular genetics of barley and rice nitrate reductases. Curr. Topics Plant Biochem. Physiol. 7 : 35-42.

McCABE D.E., SWAIN W., MARTINELLI B., CHRISTOU P. (1988) Stable transformation of soybean (Glycine max) by prticle acceleration. Bio/technology 6 : 923-927.

NEUHAUS G., SPANGENBERG G., MITTELSTEN SCHIED O., SCHWEIGER H.G. (1987) Transgenic rapeseed plants obtained by the microinjection of NDNA into microspore-derived embrioids. Theor. Appl. Genet. 75 : 30-36.

OSTREM J.A. AND COLLINS G.B. (1983) Genetic variation for nitrate concentration in Nicotiana tabacum L.J. Heredity 74 : 431-434.

ROCHA-SOSA M., SONNEWALD U., FROMMER W. STRATMAN M., SCHELL J., WILLMITYZER L. (1989) Both develomental and metabolic signals activate the promoter of a class I patatin gene. EMBO J. 8 : 23-29.

SAUX C., LEMOINE Y., MARION-POLL A., VALADIER M.H.. DENG M., MOROT-GAUDRY J.F. (1987) Consequences of absence of nitrate reductase activity on photosynthesis in Nicotiana plumbaginifolia plants. Plant. Physiol. 84 : 67-72.

STOCHER R.J., SCHILLITO R., SAUL M., PASKOWSKI J., POTRYKUS 1. (1986) Co-transformation of unlinked foreign genes into plants by direct gene transfer. Bio/technology 4 : 1093-1096.

THOMAS S.P. (1980) Hybridization of denatured RNA and small DNA fragments transferred to nitrocellulose. Proc. Natl. Acad. Sci. USA 67 : 442-447.

THOMSON W.F. AND WHITE M.J. (1991) Physiological and molecular studies of light-regulated nuclear genes in higher plants. Ann. Rev. Plant Physiol. Plant Mol. Biol. 42 : 423-466.

VAUCHERET, H., VINCENTZ, M., KRONENBERGER, J., CABOCHE, M., AND ROUZE, P. (1989) Molecular cloning and characterization of the two homeologous genes coding for nitrate reductase in tobacco. Mol. Gen. Genet. 216 : 10-15.

VINCENTZ M. et CABOCHE M. - 1991 - Constitutive expression of nitrate reductase allows normal growth and development of Nicotiana plumbaginifolia plants. EMBO J., 10, 1027-1035.

WARNER R.L. AND HUFFAKER R.C. (1989) Nitrate transport is independent of NADH and NADPH nitrate reductases in barley seedlings. Plant Physiol. 91 : 947-953.

WRAY - 1986 - The molecular genetics of higher plant nitrate assimilation - In, A genetic approach to Plant Biochemistry, A.D. Blonstein and P.J. King, eds (Springer, N.Y.), pp. 101-157.

ZAMBRYSKI P., JOOS H., GENETELLO C., LEEMANS J., VAN MONTAGU M., SCHELL J. (1983) Ti plasmid vector for the introduction of DNA into plant cells without alteration of their normal regeneration capacity EMBO J 2 : 2143-2150.

## Revendications

1. Procédé pour abaisser la teneur en nitrates stockés dans une plante, caractérisé en ce qu'on induit une surexpression de la Nitrate Réductase dans la plante.

2. Procédé selon la revendication 1, caractérisé en ce qu'il est mis en oeuvre pour accroître la précocité de la plante.

3. Procédé selon l'une des revendications 1 ou 2 caractérisé en ce qu'on introduit dans le génome de la plante un gène étranger codant pour la Nitrate Réductase dans des conditions permettant son expression.

4. Procédé selon l'une des revendications 1 à 3 caractérisé en ce que le gène codant pour la Nitrate Réductase provient d'un ADNc de plantes dicotylédones codant pour la Nitrate Réductase.

5. Procédé selon l'une des revendications 2 à 4 caractérisé en ce qu'on infecte des explants par une souche d'Agrobactérium tumefaciens ou Agrobactérium Rhizogenèse transformée par un plasmide dans lequel est inséré lesdit gène étranger codant pour la Nitrate Réductase placé sous le contrôle d'éléments assurant l'expression dudit gène.

6. Procédé selon l'une des revendications à 5, caractérisé en ce que le gène étranger codant pour la Nitrate Réductase est inséré dans un plasmide dérivé du plasmide de Ti ou Ri.

7. Procédé selon l'une des revendications 2 à 6, caractérisé en ce que le gène étranger codant pour la Nitrate Réductase est placé sous le contrôle d'un promoteur hétérologue fonctionnel dans la plante transformée.

8. Procédé selon l'une des revendications 2 à 7 caractérisé en ce que le gène étranger codant pour la Nitrate Réductase est dérivé du gène Nia 2 de la Nitrate Réductase du tabac.

9. Procédé selon l'une des revendications 2 à 7 caractérisé en ce que le gène étranger codant pour la Nitrate Réductase est placé sous le contrôle du promoteur de l'ARN 355 du Ca MV.

10. Procédé selon l'une des revendications 2 à 7 caractérisé en ce que le gène étranger codant pour la Nitrate Réductase est inséré dans le plasmide pBin 19.

## Patentansprüche

1. Verfahren zur Absenkung des Gehalts an gespeicherten Nitraten in einer Pflanze, dadurch charakterisiert, daß man eine Überexpression von Nitratreduktase in der Pflanze induziert.

2. Verfahren nach Anspruch 1, dadurch charakterisiert, daß es zur Reifesteigerung der Pflanze durchgeführt wird.

3. Verfahren nach einem der Ansprüche 1 oder 2, dadurch charakterisiert, daß man in das Genom der Pflanze ein Fremdgen einführt, das für Nitratreduktase codiert unter Bedingungen, die seine Expression ermöglichen.

4. Verfahren nach einem der Ansprüche 1 bis 3, dadurch charakterisiert, daß das für Nitratreduktase codierende Gen aus einer cDNA von dicotyledonen Pflanzen stammt, die für Nitratreduktase codiert.

5. Verfahren nach einem der Ansprüche 2 bis 4, dadurch charakterisiert, daß man Setzlinge mit einem Stamm Agrobakterium tumefaciens oder Agrobakterium Rhizogenese infiziert, der durch ein Plasmid transformiert ist, in das das für Nitratreduktase codierende Fremdgen insertiert ist, welches unter Kontrolle von Elementen steht, die die Expression dieses Gens gewährleisten.

6. Verfahren nach einem der Ansprüche 2 bis 5, dadurch charakterisiert, daß das für Nitratreduktase codierende Fremdgen in ein Plasmid insertiert ist, das vom Plasmid Ti oder Ri abgeleitet ist.

7. Verfahren nach einem der Ansprüche 2 bis 6, dadurch charakterisiert, daß das für Nitratreduktase codierende Fremdgen unter Kontrolle eines funktionellen heterologen Promoters in der transformierten Pflanze angeordnet ist.

8. Verfahren nach einem der Ansprüche 2 bis 7, dadurch charakterisiert, daß das für Nitratreduktase codierende Fremdgen vom Gen Nia 2 der Nitratreduktase aus Tabak abgeleitet ist.

9. Verfahren nach einem der Ansprüche 2 bis 7, dadurch charakterisiert, daß das für Nitratreduktase codierende Fremdgen unter Kontrolle des Promoters ARN 355 aus Ca MV angeordnet ist.

10. Verfahren nach einem der Ansprüche 2 bis 9, dadurch charakterisiert, daß das für Nitratreduktase codierende Fremdgen in das Plasmid pBin 19 insertiert ist.

## Claims

1. Method for lowering the content of nitrates stored in a plant, characterized in that an overexpression of nitrate reductase is induced in the plant.

2. Method according to Claim 1, characterized in that it is used to enhance the earliness of the plant.

3. Method according to either of Claims 1 and 2, characterized in that a foreign gene coding for nitrate reductase is introduced into the genome of the plant under conditions permitting its expression.

4. Method according to one of Claims 1 to 3, characterized in that the gene coding for nitrate reductase originates from a cDNA of dicotyledonous plants coding for nitrate reductase.

5. Method according to one of Claims 2 to 4, characterized in that explants are infected with an Agrobacterium tumefaciens or Agrobacterium Rhizogenes strain transformed with a plasmid into which is inserted the said foreign gene coding for nitrate reductase, placed under the control of elements providing for the expression of the said gene.

6. Method according to one of Claims 2 to 5, characterized in that the foreign gene coding for nitrate reductase is inserted into a plasmid derived from the Ti or Ri plasmid.

7. Method according to one of Claims 2 to 6, characterized in that the foreign gene coding for nitrate reductase is placed under the control of a heterologous promoter which is functional in the transformed plant.

8. Method according to one of Claims 2 to 7, characterized in that the foreign gene coding for nitrate reductase is derived from the Nia 2 gene for nitrate reductase of tobacco.

9. Method according to one of Claims 2 to 7, characterized in that the foreign gene coding for nitrate reductase is placed under the control of the 35S RNA promoter of Ca MV.

10. Method according to one of Claims 2 to 9, characterized in that the foreign gene coding for nitrate reductase is inserted into plasmid pBin19.
